(19) Europäisches Patentamt
European Patent Office
Office européen des brevets

(11) Veröffentlichungsnummer : **0 077 529**
**B1**

(12) **EUROPÄISCHE PATENTSCHRIFT**

(45) Veröffentlichungstag der Patentschrift : **17.07.85**

(51) Int. Cl.4 : **C 07 C 93/187**, C 07 D209/08, A 61 K 31/225, A 61 K 31/40, A 61 K 31/00, A 61 K 47/00

(21) Anmeldenummer : **82109484.4**

(22) Anmeldetag : **14.10.82**

(54) **Arzneimittelformulierung.**

(30) Priorität : **16.10.81 CH 6627/81**
**19.01.82 CH 314/82**

(43) Veröffentlichungstag der Anmeldung : **27.04.83 Patentblatt 83/17**

(45) Bekanntmachung des Hinweises auf die Patenterteilung : **17.07.85 Patentblatt 85/29**

(84) Benannte Vertragsstaaten :
**AT BE CH DE FR GB IT LI LU NL SE**

(56) Entgegenhaltungen :
**GB-A- 1 199 037**

(73) Patentinhaber : **Sanol Schwarz GmbH**
**Mittelstrasse 11-13**
**D-4019 Monheim (DE)**

(72) Erfinder : **Speiser, Peter, Prof. Dr.**
**Freudenbergerstrasse 101**
**CH-8044 Zürich (CH)**
Erfinder : **Vaizoglu, Orhan, Dr.**
**Kinkelstrasse 28**
**CH-8006 Zürich (CH)**

(74) Vertreter : **Redies, Bernd, Dr. rer. nat. et al**
**Redies, Redies, Türk & Gille Brucknerstrasse 20**
**D-4000 Düsseldorf 13 (DE)**

**EP 0 077 529 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Arzneimittelträgersystem, das eine Verabreichung des Wirkstoffs mit hoher Wirkstoffaufnahme in die Blutbahn des hiermit behandelten Patienten, insbesondere auch bei oraler Verabreichung ermöglicht. Das erfindungsgemäße Arzneimittelträgersystem besteht aus ultrafeinen Partikeln eines Umsetzungsproduktes aus einem reaktionsfähigen Derivat einer mindestens zweibasischen anorganischen Säure oder einer Alkancarbonsäure mit 2 oder 3 Carboxylgruppen und gegebenenfalls ein oder zwei Hydroxygruppen, wobei die eine Bindung der zweibasischen anorganischen Säure bzw. die eine Carboxygruppe der Alkancarbonsäure mit einem pharmakologischen Wirkstoff verbunden ist, der eine Hydroxygruppe, SH-Gruppe und/oder eine primäre oder sekundäre Aminogruppe mit einem reaktionsfähigen Wasserstoffatom an dieser Gruppe trägt, und die andere Bindung mit der freien Hydroxygruppe eines Glycerolipids mit mindestens einer freien Hydroxygruppe am Glycerin verbunden ist. Die Erfindung betrifft weiterhin diese Umsetzungsprodukte selbst sowie Verfahren zur Herstellung der ultrafeinen Partikel derselben.

Bei vielen Arzneimittelzubereitungen ist die Wirksamkeit insbesondere darum beschränkt, weil die Wirkstoffe aufgrund des hydrophilen Charakters des Wirkstoffmoleküls Organgrenzschichten nicht oder nur in geringem Umfang während der Verweilzeit an der Grenzschicht passieren können, z. B. bei oraler Verabreichung während der Passage des Magen-Darm-Traktes die hierzu gehörenden Schleimhäute. Viele Wirkstoffe enthalten im Molekül Hydroxy, SH- und/oder primäre oder sekundäre Aminogruppen, deren Wasserstoffatome reaktionsfähig sind. Bei den erfindungsgemäßen Arzneimittelträgersystemen sind die eine Hydroxygruppe, SH-Gruppe und/oder primäre oder sekundäre Aminogruppe enthaltenden Wirkstoffe über ein zwei Reaktionszentren aufweisendes, physiologisch leicht abspaltbares Brückenelement an die Hydroxygruppe eines Glycerolipids mit mindestens einer freien Hydroxygruppe gebunden, wobei die anderen Hydroxygruppen mit Resten teilweise oder ganz abgesättigt sind, die dem Gesamtmolekül einen lipophilen Charakter geben, der eine bessere Membran- oder Grenzschichtpassage ermöglicht. Durch Variation der Substituenten an den anderen Hydroxygruppen des Glycerolipids je nach Lipophilität des Wirkstoffs kann die Lipophilie des gesamten Moleküls gesteuert und somit viel besser auf optimale Membranpassage am jeweils angesteuerten Organ eingestellt werden. Die Verwendung des ausgewählten Brückenglieds zwischen Glycerolipid und Wirkstoff gewährt die leichte Freigabe des Wirkstoffs nach Passage der Grenzschicht, ohne daß bei dem Abbau Bruchstückmoleküle mit physiologisch unerwünschten Eigenschaften oder Eigenwirkung entstehen. Aufgrund ihrer lipophilen-amphiphilen Eigenschaften können die erfindungsgemäßen Verbindungen mit Wasser bzw. isotonischer Kochsalzlösung in ultrafeine disperse Suspensionen, d. h. Suspensionen von Partikeln mit einer Teilchengröße im Nanometerbereich (kleiner als 1 μm oder $10^{-3}$ mm) überführt werden, so daß weitere Hilfsstoffe außer Wasser nicht mehr notwendig sind. Die Stabilität der hierdurch hergestellten Arzneimittelzubereitungen wie auch der durch die kovalente Bindung der einzelnen Bestandteile gebildeten Prodrugs ist überraschend über lange Zeit gegeben. Dadurch, daß keine Hilfsstoffe mehr inkorporiert sind und die verwendeten Wirkstoffe nur an physiologische Lipide gebunden ist, spielt nur die Toxizität der Wirkstoffe eine Rolle. Gegenstand der Erfindung ist somit ein Arzneimittelträgersystem bestehend aus ultrafeinen Partikeln einer Verbindung der allgemeinen Formel

A—R—L

wobei A der sich nach Umsetzung des reaktiven Wasserstoffatoms sich ergebende Rest des eine oder mehrere Hydroxygruppen, SH-Gruppen und/oder primäre oder sekundäre Aminogruppen mit einem reaktionsfähigen Wasserstoffatom enthaltenden Wirkstoffs, R das Brückenelement mit zwei Reaktionszentren und L der nach Umsetzung mit der freien Hydroxygruppe sich ergebende Rest eines Glycerolipids mit mindestens einer freien Hydroxygruppe ist. Gegenstand der vorliegenden Erfindung sind weiterhin die Prodrugs der Formel

A—R—L

selbst. Enthält der Wirkstoff A—H mehrere Hydroxygruppen, SH-Gruppen und/oder primäre oder sekundäre Aminogruppen mit einem reaktionsfähigen Wasserstoffatom, können bei Einsatz entsprechender Mengen der das Brückenelement R ergebenden Reaktionskomponenten sowie der Glycerolipide mit mindestens einer freien Hydroxygruppe mehrere derartige Trägerreste —R—L mit dem Wirkstoff verbunden werden, z. B. um die angestrebte Lipophilität des Gesamtmoleküls zu erhalten.

Als Brückenelemente eignen sich insbesondere $—SO_2$, —PO(OH)—, —CO—, die Reste von Alkandicarbonsäuren, Alkantricarbonsäuren, Alkanmono- oder dihydroxydicarbonsäuren und Alkanmono- oder dihydroxytricarbonsäuren, wobei der Alkanrest 1 bis 6 Kohlenstoffatome enthalten kann. Diese Brückenelemente ergebende Reste sind z. B. Sulfurylchlorid, Phosphorsäureanhydrid, Phosgen, die Disäurechloride oder die entsprechenden Anhydride von Fumarsäure, Sebazinsäure, Malonsäure, Glutarsäure, Adipinsäure, Bernsteinsäure, Tartronsäure, Zitronensäure, Apfelsäure oder Weinsäure. Bevorzugt sind hierbei die entsprechenden Derivate der Alkandicarbonsäuren, deren Alkanrest 1 bis 3

Kohlenstoffatome enthalten und gegebenenfalls eine oder zwei Hydroxygruppen tragen kann. Ganz besonders bevorzugt ist dabei Bernsteinsäure.

Beispiele geeigneter Glycerolipide mit mindestens einer freien Hydroxygruppe am Glycerin sind Monoglyceride, Diglyceride und Phospholipide. Beispiele geeigneter Glyceride sind diejenigen, die sich von gesättigten oder ungesättigten Carbonsäuren mit 12 bis 14 Kohlenstoffatomen im Acylrest ableiten, d. h. bei denen die nicht freien Hydroxygruppen am Glycerin mit gesättigten oder ungesättigten Carbonsäuren mit 12 bis 14 Kohlenstoffatomen im Alkylrest wie z. B. Stearin-, Palmitin-, Laurin-, Myristin-, Öl-, Linol- und Linolensäure verestert sind und, bei Phospholipiden, eine Hydroxygruppe mit dem aus Phosphatidylcholin, Phosphatidylethanolamin, Phosphatidylserin und anderen natürlichen Phospholipiden bekannten Phosphorsäureresten verbunden ist.

Das erfindungsgemäße Arzneimittelträgersystem bzw. Prodrugsystem ist auf zahlreiche Arzneimittel mit einer oder mehreren Hydroxygruppen und/oder primären oder sekundären Aminogruppen mit reaktionsfähigem Wasserstoffatom hieran anwendbar. Beispiele für derartige geeignete Wirkstoffe sind :

1. Aryl- und Heteroaryloxypropanolamine

Betablocker wie :

Acebutolol
Alprenolol
Atenolol
Befunolol
Betaxolol
Bevantolol
Bometolol
Bucumolol
Bufetolol
Bufuralol
Bunitrolol
Bunolol
Bupranolol
Carazolol
Carteolol
Celiprolol
Chloropractolol
Cloranolol
H 87/07
Indenolol
Metipranol
Mepindolol
Metoprolol
Moprolol
Medroxalol
Nafetolol
Nifenalol
Nadolol
Oxprenolol
Primidolol
Practolol
Propafenon
Pamatolol
Penbutolol
Pindolol
Propranolol
Talinolol
Tazolol
Timolol
Tolamolol
Trimepranol

2. Vasodilatatoren

a) Hydrazinophthalazine und -pyridazine wie

Hydralazin
Dihydralazin
ISF 2123

b) Calciumantagonisten mit Dihydropyridincarbonsäureesterstruktur wie
Nifedipin
Nimodipin
Nisoldipin
Nicardipin
Niludipin
Ryosidin
Nitrendipin
SKF 24260
Hässle H 154/82

c) andere chemisch nicht näher klassifizierte Wirkstoffe mit reaktiven Gruppen einschließlich umesterbaren Carbonsäureestern (Acylaten) von pharmakologisch aktiven Substanzen wie
Dopamin
Dobutamin
Methyldopa
Guanethidin
Prazosin
Diltiazem (Umesterung)
Prenylamin
Phentolamin
Captopril
Teprotid
Trimazosin
Exifon
Flecainid
Procainamid
Chinidin
Tocainid
Ajmalin
Prajmalin
Hydroxytriamteren
Furosemid
Spironolacton
Amilorid
Chlortalidon
Xipamid
Benzaron
Benzbromaron
Benziodaron

d) Thiazid-Diuretika wie
Hydrochlorothiazid
Trichlormethiazid
Cyclopenthiazid
Polythiazid
Butizid
Bemetizid
Hydroflumethiazid
Ethiazid
Mebutizid
Cyclothiazid
Benzylhydrochlorothiazid
Paraflutizid
Epitizid
Methyclothiazid
Bendroflumethiazid

3. Aryl- und Heteroarylethanolamin-Derivate einschließlich Katecholamin wie
Labetalol
YM-09, 538
Sulfinanol
Salbutamol
Terbutalin
Fenoterol

Nifenalol
Clenbuterol
Bamethan
Buphenin
Isoxuprin
Zinterol
Norfenefrin
Etilefrin
Octopamin
Synephrin
Phenylephrin
TA-064
Ephedrin
Orciprenalin
Isoprenalin
Dichlorisoprenalin
Adrenalin
Noradrenalin

4. Histamin-$H_2$-Antagonisten

a) vom Cyanoguanidin-Typ wie
Cimetidin
CRC-1970
Etindidin
Impromidin
Oxmetidin
Tiotidin

b) vom Ethendiamin-Typ wie
Ranitidin

5. Prostaglandine, Prostacycline, Leukotriene

6. Herzwirksame Glycoside wie

Digitoxin
Digoxin
Methyldigoxin
Digitoxigenin
Gitoxigenin
Digoxigenin
Methyldigoxin
α- und β-Acetyldigoxin
Proscillardin
k-Strophantin

7. Diuretika vom Thiazid-Typ wie
Chlorothiazid
Flumethiazid
Hydrochlorothiazid
Benzthiazid
Trichlormethiazid
Cyclopenthiazid
Thiabuthiazid
Polythiazid
Butizid
Bemetizid
Hydroflumethiazid

8. Mutterkornalkaloide wie
Ergotamin
Ergosin
Methylergometrin
Ergocristin

Ergocryptin
Ergocornin
Methysergid

a) Hydrierte Mutterkornalkaloide wie
Dihydroergotamin
Dihydroergocornin
Dihydroergocristin
Dihydroergocryptin

9. Heparin, Heparinoide

10. Insulin

11. Chlorambucil
Actinomycin D
4-Aminopteroylglutaminsäure
Halogenhaltige Röntgenkontrastmittel mit reaktiven Gruppen

12. Steroidhormone wie
Testosteron
Progesteron
Pregnenolon
Corticosteron
Cortisol
17α-Hydroxyprogesteron
Cortison
Prednison
Prednisolon
Triamfinolon
Methylprednisolon
Fluocortolon
Dexamethason.

Die vorstehend beispielhaft aufgeführten geeigneten Wirkstoffe sind mit ihren allgemein bekannten Generic-Bezeichnungen angegeben.
Umfaßt sind als Wirkstoffe somit

a) Hydroxyverbindungen, z. B.
aliphatische Alkohole, Phenole und Enole

b) Verbindungen der SH-Gruppen, z. B.
Mercaptane und Thiophenole

c) Verbindungen mit Aminogruppen, z. B.
primäre und sekundäre Amine, Hydrazine, Harnstoffe, Guanidine, Aminoguanidine, Semicarbazide, Carbohydrazide, Thioharnstoffe, Thiosemicarbazide, Thiocarbohydrazide und Formazane.

Bevorzugt ist das erfindungsgemäße Arzneimittelträgersystem auf die Verbindungen der vorstehenden Gruppen 1, 2a-2c, 3, 5 und 6, insbesondere die Gruppen 1, 2b und 3, anwendbar, so daß A ganz besonders bevorzugt ein Aryl- oder Heteroarylethanolamin oder Aryl- oder Heteroaryloxypropanolamin oder deren Hydroxy- oder primäre oder sekundäre Aminogruppe im Ethanolamin- bzw. Propanolaminteil des Moleküls mit dem reaktionsfähigen Derivat der das Brückenglied R ergebenden Säure reagiert.
Das Verfahren zur Herstellung der erfindungsgemäßen ultrafeinen Partikel im Nanometerbereich besteht darin, daß man die Prodrugs

a) in einem mit Wasser mischbaren organischen Lösungsmittel löst und durch Zugabe von gegebenenfalls ein Tensid enthaltendem Wasser oder wässrige Pufferlösung ausfällt, die so hergestellten Partikel von der flüssigen Phase abtrennt, wäscht und wieder in einem geeigneten Medium redispergiert oder

b) in einem flüchtigen Lösungsmittel, gegebenenfalls zusammen mit einem geeigneten Tensid löst, das Lösungsmittel abdampft und den Rückstand in einem geeigneten Dispergiermittel wie Wasser, Pufferlösung oder einem geeigneten mit Wasser oder Pufferlösung mischbaren organischen Lösungsmittel (Alkohole, Aceton o. ä.) durch kräftiges Rühren und ggfs. unter Ultraschalleinwirkung ultrafein dispergiert oder

c) bis 5 °C über ihre Schmelzpunkte erhitzt, die Schmelze tropfenweise unter kräftigem Rühren, gegebenenfalls unter Ultraschall-Einwirkung, in gegebenenfalls ein Tensid enthaltendes Wasser einträgt, dessen Temperatur der Temperatur der Schmelze entspricht und die erhaltene wässrige Lösung schnell abkühlt.

In den Verfahren kann anstelle von Wasser jeweils eine übliche Puffer-Lösung verwendet werden. Infragekommende Lösungsmittel für die Stufe a) sind z. B. Dioxan, Dimethylsulfoxid (DMSO), Tetrahydrofuran, aliphatische Alkohole, Aceton und andere Ketone oder Gemische der genannten Lösungsmittel.

Infragekommende Tenside sind u. a. Tweens, Spans, Pluoronics, Lecithine und, je nach Applikationsart, alle oberflächenaktiven Tensid-Klassen wie anionische, kationische und nonionische Tenside.

Als flüchtige Lösungsmittel der Verfahrensvariante b) kommen z. B. Chloroform, Methylenchlorid, Toluol, Hexan, Dichloräthan infrage. Die verwendeten Tenside sind die in der Verfahrensvariante a) angegebenen Verbindungen. Sie gelten auch für die Verfahrensvariante c).

Die hergestellten Prodrugs zeigen, je nach chemischer Struktur, starke oder weniger starke Oberflächenaktivität. Ob für die Herstellung der ultrafeinen Partikel überhaupt Tenside eingesetzt werden müssen oder nicht, hängt von der chemischen Struktur des Prodrugs ab.

Die so hergestellten Arzneimittelträgersysteme können peroral appliziert werden, wo sie im gastrointestinalen Trakt durch Pinocytose oder durch andere passive Adsorptionsmechanismen aufgenommen werden. Sie können auch parenteral appliziert werden.

Geeignete Darreichungsformen sind z. B. Tabletten, Kapseln, Pflaster, ultrafeine Suspensionen, Implantationen, Salben, Nasentropfen für systemische Wirkung und dergleichen, die nach herkömmlichen Methoden hergestellt werden.

Die erfindungsgemäßen Prodrugs zeichnen sich durch
— hohe Lipophilie
— Oberflächenaktivität
— gute biologische Verträglichkeit
— langsame enzymatische Abbaugeschwindigkeit, die gleichzeitig eine Depotwirkung und Verminderung des « First-Pass-Effektes » ermöglicht sowie
— vielfältige galenische Verarbeitungsmöglichkeiten aus.

So können sie z. B. hergestellt werden aus Wirkstoffen, die nach peroraler gabe schlechte Resorptionseigenschaften zeigen oder einen hohen « First-Pass-Effekt » aufweisen. Auch Wirkstoffe, die schwer löslich sind, sind für die Bildung von Prodrugs geeignet.

Die erfindungsgemäßen Prodrugs sind neu und sind somit ebenfalls gegenstand der vorliegenden Erfindung.

Die Herstellung der Prodrugs geschieht unter Anwendung an sich bekannter Verfahren zur Herstellung von Estern, Thioestern bzw. Amiden oder zur Umesterung von Estern ausgehend von reaktiven Derivaten der Molekülbruchstücke A, R und L oder der Molekülbruchstück-Verbindungen selbst unter Anwendung üblicher Reaktionsbedingungen.

Die nachfolgenden Beispiele veranschaulichen die Erfindung. Temperaturangaben beziehen sich auf Celsiusgrade.

## Beispiel 1

a) Herstellung des Bernsteinsäure-Monoesters des 1,3-Dipalmitins

30 g (0,052 7 mol) 1,3-Dipalmitylglycerin und 45 g (0,447 mol) Bernsteinsäureanhydrid werden in 750 ml wasserfreiem Benzol suspendiert und die Mischung in Gegenwart von 7,5 ml trockenem Pyridin unter Rückfluß und Wasserausschluß gekocht. Nach 25 Std. wird das Reaktionsgemisch abgekühlt, filtriert und bis zu einem wachsartigen Feststoff eingedampft. Dieses Produkt wird mit kochendem Wasser behandelt und nach dem Abkühlen mit Chloroform extrahiert. Die Chloroformschicht wird mit 0,1 N HCl und Wasser gewaschen, über $MgSO_4$ getrocknet und eingedampft. Das feste Produkt wird mit Aceton/Heptan/Ammoniak (25 %) (75 : 25 : 3) zerrieben und der Niederschlag abfiltriert und mit Aceton/Heptan (60 : 40) gewaschen. Nach Isolierung wird der Niederschlag in Chloroform gelöst, mit 0,1 N HCl und Wasser gewaschen und die Chloroformlösung über $MgSO_4$ getrocknet und eingedampft. Nach Umkristallisierung aus Aceton erhält man das Titelprodukt.

b) Herstellung des 1,3-Dipalmitylbernsteinsäure-Monoesteracylchlorids

30 g (0,045 mol) 1,3-Dipalmitin-Bernsteinsäuremonoester werden mit 2 Tropfen N,N-Dimethylformamid in 100 ml trockenem Methylenchlorid gelöst. Das Gemisch wird auf 5° gekühlt, 3 ml Thionylchlorid langsam tropfenweise zugegeben und das Ganze 3 Stunden lang unter Rückfluß und Wasserausschluß

gekocht. Das Lösungsmittel wird abgedampft und das Titelprodukt aus n-Heptan umkristallisiert.

c) Veresterung von Bupranolol-HCl

11,0 g (0,016 mol) 1,3-Dipalmitin-Bernsteinsäuremonoesteracylchlorid werden in 350 ml Chloroform gelöst, auf 5° gekühlt und 1,6 g trockenes Pyridin zugegeben. Anschließend wird während einer Stunde bei 5° eine Suspension von 6,2 g Bupranolol-HCl in trockenem Chloroform tropfenweise zugegeben. Das Reaktionsgemisch wird dann während 48 Stunden unter Rückfluß und Wasserausschluß bei Raumtemperatur weiterbehandelt. Die Chloroform-Lösung wird anschließend mit 0,1 N HCl und Wasser gewaschen, über $MgSO_4$ getrocknet und abgedampft. Das so erhaltene gummiartige Produkt wird durch Säulenchromatographie gereinigt. Das Produkt kann gegebenenfalls analog Beispiel 1 weiterverarbeitet werden.

| Elementaranalyse | C | H | N |
|---|---|---|---|
| Berechnet | 68,99 | 10,05 | 1,52 |
| Gefunden | 69,02 | 10,15 | 1,53 |

Beispiel 2

140 mg des gemäß Beispiel 1 hergestellten Prodrugs werden mit 250 mg Tween 80 in 20 ml Chloroform gelöst, das Chloroform wird im Rotationsverdampfer abgedampft, und dem Rückstand werden 50 ml Phosphatpuffer pH 7 zugegeben und die Suspension unter kräftigem Rühren unter Ultraschall-Einwirkung 10 Minuten behandelt. Es entstehen Partikel von 160 nm (= Nanometer).

Beispiel 3

140 mg des gemäß Beispiel 1 hergestellten Prodrugs werden bei 60° geschmolzen und in 50 ml 0,4 %-iger Tween 80-Lösung in Phosphatpuffer pH 7, die ebenfalls bei 60° temperiert ist, unter heftigem Rühren tropfenweise zugegeben, 10 Minuten mit Ultraschall behandelt. Die entstehende ultrafeine Emulsion wird langsam abgekühlt, wobei sie sich in eine ultrafeine Suspension umwandelt.

Beispiel 4

140 mg des gemäß Beispiel 1 hergestellten Prodrugs werden in einem Gemisch von 7 ml Dioxan + 30 ml DMSO + 100 mg Tween 80 gelöst. Diese Lösung wird unter schnellem Rühren in 50 ml 0,4 %-iger Tween-Lösung in Phosphatpuffer pH 7 allmählich zugetropft. Die Partikelgröße wird sich je nach Verarbeitungstechnik und verwendeten Substanzen und anderen Proportionen ändern. Meistens bewegt sich die Partikelgröße im Bereich von 0,1-1 µm.

Beispiel 5

200 mg Pindolol-Prodrug entsprechend Beispiel 1, hergestellt durch Verwendung der entsprechenden Menge von Pindolol-HCl anstelle von Bupranolol-HCl im Beispiel 1, und 500 mg Tween 80 werden in Chloroform gelöst und das Chloroform abgedampft. Der Rückstand wird in 70,67 ml 0,9 %-iger NaCl-Lösung unter Ultraschall-Einwirkung dispergiert, was 2,83 mg/ml · kg Pindolol-Prodrug und 1 mg/ml · kg Pindolol entspricht. Die durchschnittliche Teilchengröße der Partikel betrug 25 bis 50 nm.

Beispiel 6

Anwendungsbeispiele

4 Hunden wurden in einer randomisierten Versuchsreihe die folgenden Formen i. v. bzw. p. o. in Crossover-Versuchen verabreicht.

Form A: Pindolol-HCl, 1 mg/ml · kg in 0,9 %-iger NaCl-Lösung

Form B: Arzneimittelzubereitung gemäß Beispiel 5

Analytik: Es wurden je 5 ml Blut entnommen, heparinisiert, zentrifugiert und das Plasma bis zur Analyse bei − 20° aufbewahrt. Die Bestimmung erfolgte größtenteils in einer für unverändertes Pindolol spezifischen, fluorometrischen Methode auf einem Ismatec-Analysenautomaten (W. Pacha, Progress in Quality control at Medicines. Deasy and Zimoney (Eds.), Elsevier Biomedical Press 1981, Automated analysis of pharmacokinetic studies, S. 155-192). Zur Kontrolle wurden einige Proben auch mit einer HPLC-Methode und fluorometrischer Detektion reanalysiert (W. Pacha, M. Feltin, wird noch veröffentlicht).

Ergebnisse

1. Eine Abschätzung des Leber-First-Pass-Effektes aus der $AUC_{iv}$ ergibt nach peroraler Gabe beim erfindungsgemäßen Produkt einen Wert von nur 30 %, wobei er beim Pindolol-Wirkstoff 70 % beträgt. Die

Vermutung, daß durch das erfindungsgemäße Prodrug der Leber-First-Pass-Effekt vermindert werden könnte, ist somit bestätigt.

2. Intravenös wird nach Verabreichung des erfindungsgemäßen Produktes ein etwa 3 mal höherer Plasmaspiegel gefunden als nach Verabreichung der unverändertes Pindolol enthaltenden Lösung.

**Patentansprüche** (für die Vertragsstaaten : BE, CH, DE, FR, GB, IT, LI, LU, NL, SE)

1. Arzneimittelträgersystem, bestehend aus ultrafeinen Partikeln einer Verbindung der Formel

A—R—L

wobei A der Rest eines eine oder mehrere Hydroxygruppen, SH-Gruppen und/oder primäre oder sekundäre Aminogruppen mit einem reaktionsfähigen Wasserstoffatom enthaltenden pharmakologischen Wirkstoffes ist, der sich nach Umsetzung des reaktionsfähigen Wasserstoffatoms an der oder den Hydroxygruppen, SH-Gruppen und/oder Aminogruppen ergibt, R der den Rest einer mindestens zweibasischen anorganischen Säure oder einer Alkancarbonsäure mit zwei oder drei Carboxylgruppen und gegebenenfalls ein oder zwei Hydroxylgruppen im Molekül und mit 1 bis 6 Kohlenstoffatomen im Alkanrest ist und L der Rest eines Glycerolipids mit einer oder zwei freien Hydroxylgruppen am Glycerinteil des Moleküls ist, und gegebenenfalls üblichen inerten galenischen Trägerstoffen und/oder Lösungsmitteln.

2. Arzneimittelträgersystem gemäß Anspruch 1, dadurch gekennzeichnet, daß R der Bernsteinsäurerest —CO—$CH_2$—$CH_2$—CO— ist.

3. Die Prodrug-Verbindungen der allgemeinen Formel

A—R—L

wobei A der Rest eines eine oder mehrere Hydroxygruppen, SH-Gruppen und/oder primäre oder sekundäre Aminogruppen mit einem reaktionsfähigen Wasserstoffatom enthaltenden pharmakologischen Wirkstoffs ist, der sich nach Umsetzung des reaktionsfähigen Wasserstoffatoms an der oder den Hydroxygruppen, SH-Gruppen und/oder Aminogruppen ergibt, R der den Rest einer mindestens zweibasischen anorganischen Säure oder einer Alkancarbonsäure mit zwei oder drei Carboxylgruppen und gegebenenfalls einer oder zwei Hydroxylgruppen im Molekül und mit 1 bis 6 Kohlenstoffatomen im Alkanrest ist und L der Rest eines Glycerolipids mit einer oder zwei freien Hydroxylgruppen am Glycerinteil des Moleküls ist.

4. Die Prodrug-Verbindung gemäß Anspruch 3, dadurch gekennzeichnet, daß R der Bernsteinsäurerest —CO—$CH_2$—$CH_2$—CO— ist.

5. Verfahren zur Herstellung des Arzneimittelträgersystems gemäß Anspruch 1, dadurch gekennzeichnet, daß man eine Prodrug-Verbindung der allgemeinen Formel

A—R—L

wobei A der Rest eines eine oder mehrere Hydroxygruppen, SH-Gruppen und/oder primäre oder sekundäre Aminogruppen mit einem Reeaktionsfähigen Wasserstoffatom enthaltenden pharmakologischen Wirkstoffs ist, der sich nach Umsetzung des reaktionsfähigen Wasserstoffatoms an der oder den Hydroxygruppen, SH-Gruppen und/oder Aminogruppen ergibt, R der den Rest einer mindestens zweibasischen anorganischen Säure oder einer Alkancarbonsäure mit zwei oder drei Carboxylgruppen und gegebenenfalls einer oder zwei Hydroxylgruppen im Molekül und mit 1 bis 6 Kohlenstoffatomen im Alkanrest ist und L der Rest eines Glycerolipids mit einer oder zwei freien Hydroxylgruppen am Glycerinteil des Moleküls ist,

a) in einem mit Wasser mischbaren organischen Lösungsmittel löst und durch Zugabe von gegebenenfalls ein Tensid enthaltendes Wasser oder wässrige Pufferlösung ausfällt, die so hergestellten Partikel von der flüssigen Phase abtrennt, wäscht und wieder in einem geeigneten Medium redispergiert oder

b) in einem flüchtigen Lösungsmittel, gegebenenfalls zusammen mit einem geeigneten Tensid löst, das Lösungsmittel abdampft und den Rückstand in einem geeigneten Dispergiermittel oder einem geeigneten mit Wasser oder Pufferlösung mischbaren organischen Lösungsmittel durch kräftiges Rühren ultrafein dispergiert oder

c) bis 5 °C über ihre Schmelzpunkte erhitzt, die Schmelze tropfenweise unter kräftigem Rühren, gegebenenfalls unter Ultraschall-Einwirkung, in gegebenenfalls ein Tensid enthaltendes Wasser zugibt, dessen Temperatur der Temperatur der Schmelze entspricht und die erhaltene wässrige Lösung abkühlt.

**Patentansprüche** (für den Vertragsstaat AT)

1. Verfahren zur Herstellung eines Arzneimittelträgersystems bestehend aus ultrafeinen Partikeln

einer Verbindung der Formel

A—R—L

wobei A der Rest eines eine oder mehrere Hydroxygruppen, SH-Gruppen und/oder primäre oder sekundäre Aminogruppen mit einem reaktionsfähigen Wasserstoffatom enthaltenden pharmakologischen Wirkstoffes ist, der sich nach Umsetzung des reaktionsfähigen Wasserstoffatoms an der oder den Hydroxygruppen, SH-Gruppen und/oder Aminogruppen ergibt, R der den Rest einer mindestens zweibasischen anorganischen Säure oder einer Alkancarbonsäure mit zwei oder drei Carboxylgruppen und gegebenenfalls ein oder zwei Hydroxylgruppen im Molekül und mit 1 bis 6 Kohlenstoffatomen im Alkanrest ist und L der Rest eines Glycerolipids mit einer oder zwei freien Hydroxylgruppen am Glycerinteil des Moleküls ist, und gegebenenfalls üblichen inerten galenischen Trägerstoffen und/oder Lösungsmitteln, dadurch gekennzeichnet, daß man eine Prodrug-Verbindung der allgemeinen Formel

A—R—L

wobei A der Rest eines eine oder mehrere Hydroxygruppen, SH-Gruppen und/oder primäre oder sekundäre Aminogruppen mit einem reaktionsfähigen Wasserstoffatom enthaltenden pharmakologischen Wirkstoffs ist, der sich nach Umsetzung des reaktionsfähigen Wasserstoffatoms an der oder den Hydroxygruppen, SH-Gruppen und/oder Aminogruppen ergibt, R der den Rest einer mindestens zweibasischen anorganischen Säure oder einer Alkancarbonsäure mit zwei oder drei Carboxylgruppen und gegebenenfalls einer oder zwei Hydroxylgruppen im Molekül und mit 1 bis 6 Kohlenstoffatomen im Alkanrest ist und L der Rest eines Glycerolipids mit einer oder zwei freien Hydroxylgruppen am Glycerinteil des Moleküls ist,

a) in einem mit Wasser mischbaren organischen Lösungsmittel löst und durch Zugabe von gegebenenfalls ein Tensid enthaltendes Wasser oder wässrige Pufferlösung ausfällt, die so hergestellten Partikel von der flüssigen Phase abtrennt, wäscht und wieder in einem geeigneten Medium redispergiert oder

b) in einem flüchtigen Lösungsmittel, gegebenenfalls zusammen mit einem geeigneten Tensid löst, das Lösungsmittel abdampft und den Rückstand in einem geeigneten Dispergiermittel oder einem geeigneten mit Wasser oder Pufferlösung mischbaren organischen Lösungsmittel durch kräftiges Rühren ultrafein dispergiert oder

c) bis 5 °C über ihre Schmelzpunkte erhitzt, die Schmelze tropfenweise unter kräftigem Rühren, gegebenenfalls unter Ultraschall-Einwirkung, in gegebenenfalls ein Tensid enthaltendes Wasser zugibt, dessen Temperatur der Temperatur der Schmelze entspricht und die erhaltene wässrige Lösung abkühlt.

2. Verfahren zur Herstellung eines Arzneimittelträgersystems gemäß Anspruch 1, dadurch gekennzeichnet, daß R der Bernsteinsäurerest $—CO—CH_2—CH_2—CO—$ ist.

**Claims** (for the Contracting States : BE, CH, DE, FR, GB, IT, LI, LU, NL, SE)

1. Pharmaceutical vehicle system comprising ultrafine particles of a compound of the formula

A—R—L

wherein A is the residue of a pharmacological active ingredient containing one or more hydroxy groups, SH groups and/or primary or secondary amino groups having a reactive hydrogen atom, which is obtained following the reaction of the reactive hydrogen atom on the hydroxy group or groups, SH groups and/or amino groups, R is the residue of an at least dibasic inorganic acid or an alkane-carboxylic acid having two or three carboxyl groups and optionally one or two hydroxyl groups in the molecule and having 1 to 6 carbon atoms in the alkane residue, and L is the residue of a glycerolipid having one or two free hydroxyl groups on the glycerol part of the molecule, and optionally conventional, inert galenic carrier substances and/or solvents.

2. Pharmaceutical vehicle system according to Claim 1, characterized in that R is the succinic acid residue $—CO—CH_2—CH_2—CO—$.

3. The prodrug compounds of the general formula

A—R—L

wherein A is the residue of a pharmacological active ingredient containing one or more hydroxy groups, SH groups and/or primary or secondary amino groups having a reactive hydrogen atom, which is obtained following the reaction of the reactive hydrogen atom on the hydroxy group or groups, SH groups and/or amino groups, R is the residue of an at least dibasic inorganic acid or of an alkane-carboxylic acid having one or two carboxyl groups and optionally one or two hydroxyl groups in the molecule, and having

1 to 6 carbon atoms in the alkane radical, and L is the residue of a glycerolipid having one or two hydroxyl groups on the glycerol part of the molecule.

4. The prodrug compound according to Claim 3, characterized in that R is the succinic acid radical $-CO-CH_2-CH_2-CO-$.

5. Process for the preparation of the pharmaceutical vehicle system according to Claim 1, characterized in that a prodrug compound of the general formula

$$A-R-L$$

wherein A is the residue of a pharmacological active ingredient containing one or more hydroxy groups, SH groups and/or primary amino groups having a reactive hydrogen atom, which is obtained following the reaction of the reactive hydrogen atom on the hydroxy group or groups, SH groups and/or amino groups, R is the residue of an at least dibasic inorganic acid or an alkane-carboxylic acid having two or three carboxyl groups and optionally one or two hydroxyl groups in the molecule, and having 1 to 6 carbon atoms in the alkane radical, and L is the residue of a glycerolipid having one or two free hydroxyl groups on the glycerol part of the molecule,

a) is dissolved in an organic solvent miscible with water and precipitated by the addition of water optionally containing a surfactant, or an aqueous buffer solution, the particles produced in this manner separated from the liquid phase, washed and redispersed in a suitable medium, or

b) is dissolved in a volatile solvent, optionally together with a suitable surfactant, the solvent evaporated and the residue dispersed in an ultrafine manner in a suitable dispersing medium or a suitable organic solvent miscible with water or a buffer solution, under vigorous agitation, or

c) is heated to up to 5° above its melting point, the melt added dropwise with vigorous agitation, optionally under the effect of ultrasonic radiation, to water optionally containing a surfactant, with the temperature of said water corresponding to the temperature of the melt, and the aqueous solution obtained cooled.

**Claims** (for the Contracting State AT)

1. Process for the preparation of a pharmaceutical vehicle system comprising ultrafine particles of a compound of the formula

$$A-R-L$$

wherein A is the residue of a pharmacological active ingredient containing one or more hydroxy groups, SH groups and/or primary or secondary amino groups having a reactive hydrogen atom, which is obtained following the reaction of the reactive hydrogen atom on the hydroxy group or groups, SH groups and/or amino groups, R is the residue of an at least dibasic inorganic acid or an alkane-carboxylic acid having two or three carboxyl groups and optionally one or two hydroxyl groups in the molecule and having 1 to 6 carbon atoms in the alkane residue, and L is the residue of a glycerolipid having one or two free hydroxyl groups on the glycerol part of the molecule, and optionally conventional, inert galenic carrier substances and/or solvents characterized in that a prodrug compound of the general formula

$$A-R-L$$

wherein A is the residue of a pharmacological active ingredient containing one or more hydroxy groups, SH groups and/or primary amino groups having a reactive hydrogen atom, which is obtained following the reaction of the reactive hydrogen atom on the hydroxy group or groups, SH groups and/or amino groups, R is the residue of an at least dibasic inorganic acid or an alkane-carboxylic acid having two or three carboxyl groups and optionally one or two hydroxyl groups in the molecule, and having 1 to 6 carbon atoms in the alkane radical, and L is the residue of a glycerolipid having one or two free hydroxyl groups on the glycerol part of the molecule,

a) is dissolved in an organic solvent miscible with water and precipitated by the addition of water optionally containing a surfactant, or an aqueous buffer solution, the particles produced in this manner separated from the liquid phase, washed and redispersed in a suitable medium, or

b) is dissolved in a volatile solvent, optionally together with a suitable surfactant, the solvent evaporated and the residue dispersed in an ultrafine manner in a suitable dispersing medium or a suitable organic solvent miscible with water or a buffer solution, under vigorous agitation, or

c) is heated to up to 5° above its melting point, the melt added dropwise with vigorous agitation, optionally under the effect of ultrasonic radiation, to water optionally containing a surfactant, with the temperature of said water corresponding to the temperature of the melt, and the aqueous solution obtained cooled.

2. Process according to claim 1 characterized in that R is the succinic acid residue $-CO-CH_2-CH_2-CO-$.

**Revendications** (pour les Etats contractants : BE, CH, DE, FR, GB, IT, LI, LU, NL, SE)

1. Système support médicamenteux constitué de particules ultrafines d'un composé de formule

A—R—L

où A représente le radical d'une substance active pharmacologique contenant un ou plusieurs groupes hydroxy, groupes SH et/ou groupes amino primaires ou secondaires avec un atome d'hydrogène réactif, qui résulte de la réaction de l'atome d'hydrogène réactif sur le ou les groupes hydroxy, groupes SH et/ou groupes amino, R représente le radical d'un acide inorganique au moins dibasique ou d'un acide alcanecarboxylique avec 2 ou 3 groupes carboxyle et éventuellement 1 ou 2 groupes hydroxyle dans la molécule et avec de 1 à 6 atomes de carbone dans le radical alcane et L est le radical d'un glycérolipide avec 1 ou 2 groupes hydroxyle libres sur la fraction glycérine de la molécule, et éventuellement des supports et/ou solvants galéniques inertes habituels.

2. Système de support médicamenteux selon la revendication 1, caractérisé en ce que R est le radical acide succinique $—CO—CH_2—CH_2—CO—$.

3. Les composés prodrug de formule générale

A—R—L

où A est le radical d'une substance active pharmacologique contenant un ou plusieurs groupes hydroxy, groupes SH et/ou groupes amino primaires ou secondaires avec un atome d'hydrogène réactif, qui provient de la réaction de l'atome d'hydrogène réactif sur le ou les groupes hydroxy, groupes SH et/ou groupes amino, R représente le radical d'un acide inorganique au moins dibasique ou d'un acide alcanecarboxylique avec 2 ou 3 groupes carboxyle et éventuellement 1 ou 2 groupes hydroxyle dans la molécule et avec de 1 à 6 atomes de carbone dans le radical alcane et L est le radical d'un glycérolipide avec 1 ou 2 groupes hydroxyle libres sur la fraction glycérine de la molécule.

4. Le composé prodrug selon la revendication 3, caractérisé en ce que R est le radical acide succinique $—CO—CH_2—CH_2—CO—$.

5. Procédé de préparation du système support médicamenteux selon la revendication 1, caractérisé en ce qu'on traite un composé prodrug de formule générale

A—R—L

où A est le radical d'une substance active pharmacologique contenant un ou plusieurs groupes hydroxy, groupes SH et/ou groupes amino secondaires avec un atome d'hydrogène réactif, qui dérive de la réaction de l'atome d'hydrogène réactif sur le ou les groupes hydroxy, groupes SH et/ou groupes amino, R représente le radical d'un acide inorganique au moins dibasique ou d'un acide alcanecarboxylique avec 2 ou 3 groupes carboxyle et éventuellement 1 ou 2 groupes hydroxyle dans la molécule et avec de 1 à 6 atomes de carbone dans le radical alcane et L est le radical d'un glycérolipide avec 1 ou 2 groupes hydroxyle libres sur la fraction glycérine de la molécule, de la façon suivante

a) on dissout dans un solvant organique miscible à l'eau et on précipite par addition d'eau ou d'une solution tampon aqueuse contenant éventuellement un agent tensio-actif, on sépare de la phase liquide les particules ainsi préparées, on lave et on redisperse dans un milieu approprié ou

b) on dissout dans un solvant volatil, éventuellement avec un agent tensio-actif approprié, on fait évaporer le solvant et on disperse le résidu dans un dispersant approprié ou un solvant organique approprié miscible à l'eau ou à une solution tampon en agitant fortement pour obtenir une dispersion ultrafine ou

c) on chauffe jusqu'à 5 °C au-dessus du point de fusion, on introduit goutte à goutte la masse fondue en agitant fortement, éventuellement sous l'action d'ultra-sons, dans de l'eau contenant éventuellement un agent tensio-actif, dont la température correspond à la température de la masse fondue et on refroidit la solution aqueuse obtenue.

**Revendications** (pour l'Etat contractant AT)

1. Procédé de préparation d'un système support médicamenteux constitué de particules ultrafines d'un composé de formule

A—R—L

où A représente le radical d'une substance active pharmacologique contenant un ou plusieurs groupes hydroxy, groupes SH et/ou groupes amino primaires ou secondaires avec un atome d'hydrogène réactif, qui résulte de la réaction de l'atome d'hydrogène réactif sur le ou les groupes hydroxy, groupes SH et/ou groupes amino, R représente le radical d'un acide inorganique au moins dibasique ou d'un acide

alcanecarboxylique avec 2 ou 3 groupes carboxyle et éventuellement 1 ou 2 groupes hydroxyle dans la molécule et avec de 1 à 6 atomes de carbone dans le radical alcane et L est le radical d'un glycérolipide avec 1 ou 2 groupes hydroxyle libres sur la fraction glycérine de la molécule, et éventuellement des supports et/ou solvants galéniques inertes habituels caractérisé en ce qu'on traite un composé prodrug de formule générale

A—R—L

où A est le radical d'une substance active pharmacologique contenant un ou plusieurs groupes hydroxy, groupes SH et/ou groupes amino secondaires avec un atome d'hydrogène réactif, qui dérive de la réaction de l'atome d'hydrogène réactif sur le ou les groupes hydroxy, groupes SH et/ou groupes amino, R représente le radical d'un acide inorganique au moins dibasique ou d'un acide alcanecarboxylique avec 2 ou 3 groupes carboxyle et éventuellement 1 ou 2 groupes hydroxyle dans la molécule et avec de 1 à 6 atomes de carbone dans le radical alcane et L est le radical d'un glycérolipide avec 1 ou 2 groupes hydroxyle libres sur la fraction glycérine de la molécule, de la façon suivante

a) on dissout dans un solvant organique miscible à l'eau et on précipite par addition d'eau ou d'une solution tampon aqueuse contenant éventuellement un agent tensio-actif, on sépare de la phase liquide les particules ainsi préparées, on lave et on redisperse dans un milieu approprié ou

b) on dissout dans un solvant volatil, éventuellement avec un agent tensio-actif approprié, on fait évaporer le solvant et on disperse le résidu dans un dispersant approprié ou un solvant organique approprié miscible à l'eau ou à une solution tampon en agitant fortement pour obtenir une dispersion ultrafine ou

c) on chauffe jusqu'à 5 °C au-dessus du point de fusion, on introduit goutte à goutte la masse fondue en agitant fortement, éventuellement sous l'action d'ultra-sons, dans de l'eau contenant éventuellement un agent tensio-actif, dont la température correspond à la température de la masse fondue et on refroidit la solution aqueuse obtenue.

2. Procédé selon la revendication 1, caractérisé en ce que R est le radical acide succinique $—CO—CH_2—CH_2—CO—$.